# EUROPEAN PATENT APPLICATION

(11) **EP 4 793 957 A1**
(43) Date of publication of application: **19.08.2026**
(21) Application number: 26156909.9
(22) Date of filing: 06.02.2026
(51) Int. Cl.: G16C 20/30, G16C 60/00, G05B 13/02, G16C 20/70, G06Q 50/04, B01F 35/82

(54) **PREDICTING TINTERS WEIGHTS TO ACHIEVE DESIRED CIELAB COLOR**

(30) Priority: 14.02.2025 IN 202511012613
(71) Applicant: Schneider Electric Systems USA, Inc., Foxborough, MA 02035 (US)
(72) Inventor: BHATTACHARYYA, Amitabha, 500019 Telangana (IN); SINHA, Bhaskar, 500019 Telangana (IN); PATIL, Ashish, 411045 Maharashtra (IN)
(74) Representative: Openshaw & Co.

(57) **Abstract**

A method of tinting liquids in an industrial mixing system. A color digitization processor receives a color measurement value and executes an artificial intelligence, Al, tinting prediction engine. The Al tinting prediction engine stores the color measurement value in a database, which contains a plurality of historical batch sample color information, a plurality of target standard sample color measurement values, and a plurality of colorant information. The Al tinting prediction engine generates predicted colorants and masses through one or more models, generates a model selection policy for the target standard sample color, and then selects predicted colorants and colorant masses based on the policy. A virtual sensor models tinting of the batch with the predicted colorants, and a predicted weight accuracy engine determines a confidence interval based on the modeled tinting and a perception boundary. The predicted colorants are transmitted to the mixing system and mixed into the liquid batch.

## Description

### BACKGROUND

Industrial tinting is the process of adjusting the color of a liquid product to match a specific target for its intended use. The conventional tinting process is lengthy, requiring multiple iterations of testing samples and adding colorants to achieve the desired color. In traditional paint tinting, a sample from a liquid batch is applied to a test panel, which is then cured to reveal the actual color. This test panel is compared to a reference panel of the target color to assess the match. A tinting expert from quality control evaluates the sample and target panels' colors to determine the appropriate colorants and their quantities to be added to the liquid batch to achieve the target color within a specified tolerance. Consequently, this process often results in significant time consumption and waste, as new test panels must be created for each evaluation.

### SUMMARY

The tinting process relies heavily on the expertise of a tinting specialist to achieve the desired results. A thorough understanding of product colors, colorants, and their respective strengths is essential for successful tinting. However, plant managers and operators often lack insight into the tinting process, including the duration of operations and the number of iterations required. This dependence on individual expertise can lead to variability in tinting outcomes, with results differing from expert to expert. Consequently, the inconsistency in the tinting process can affect batch-to-batch uniformity, making it challenging for plant managers and operators to effectively plan batch schedules.

Current conventional tools for measuring liquid color have limitations which prevent accurate measurement of color. Although spectrophotometers can determine color measurements, measuring liquids such as paints in existing systems introduce several potential sources of error. For instance, the meniscus of the liquid can affect the measurement accuracy, and freshly produced high viscous liquids such as paint often contain air bubbles that further compromise the precision of the readings.

Simple modeling of the tinting process can aid in the batch tinting process. However, many conventional models operating alone have significant limitations. Not all models operate effectively for all environments because each has various factors influencing the tinting process such as lighting and the absorption of the material. For example, the Kubelka-Munk theory provides a mathematical relationship for modeling the appearance of paint films. However, the Kubelka-Munk theory is limited in its applications. For example: the theory does not work for materials with high absorption coefficients, it relies on simplifications and assumptions that do not hold true for all materials, it requires non-negative absorption coefficients, scattering coefficients, and concentrations, and it is not suitable for complex mixtures or materials with unique optical properties.

Further, reliance on a singular model limits the ability of the tinting process to adapt. As previously mentioned, the tinting process is subject to various factors that affect the accuracy. By modeling using only one model, the process is limited to whatever constraints bind the model. Thus, the model does not learn or adapt to changing environmental factors. Further, the model does not account for the tinting expert's visual perception. A tinting expert may find a color acceptable for a given stock keeping unit (SKU) because of how the human eye perceives color, that would be outside the range of a prediction by the model. As a result, the simple model does not evolve based on the tinting expert's visual perception. Additionally, a singular model may not be suitable for all SKU's. A given SKU may operate in different environments or different materials requiring different assumptions and calculations in modeling the tinting process, which means no singular model can model the tinting process accurately for all SKU's.

Aspects of the present disclosure provide an artificial intelligence (AI) based liquid tinting solution incorporating one or more systems for accurate measurement, historical tinting information, and colorant dosing estimation to provide efficient tinting process within an industrial mixing system.

In an aspect, a method of tinting liquids in an industrial mixing system comprises receiving, at a color digitization processor, a batch color measurement value associated with a liquid batch in an industrial mixing system. The method further comprises transmitting the batch color measurement value to a color digitization processor and executing, by the color digitization processor, an Al tinting prediction engine. Executing the Al tinting prediction engine comprises storing the batch color measurement value in a color digitization database wherein the database comprises a plurality of historical batch sample color information, a plurality of target standard sample color measurement values, and a plurality of colorant information wherein the colorant information comprises a colorant value. Executing the Al tinting prediction engine further includes modeling, by one or more models, tinting of the liquid batch by weighting one or more of the batch color measurement value, a batch volume, the plurality of historical batch sample color information, or the plurality of colorant information to generate one or more predicted colorants and masses thereof from the plurality of colorant information to achieve the target standard sample color measurement. The method further comprises executing, by the color digitization processor, a virtual color sensor. Executing the virtual color sensor includes performing virtual tinting based upon the batch color measurement value, the predicted colorants, and the masses thereof and generating a predicted color coordinate range based upon the virtual tinting. The method also comprises executing, by the color digitization processor, a predicted weight accuracy engine. Executing the predicted weight accuracy engine includes generating a perception boundary based on the plurality of historical batch sample color information and the plurality of target standard sample color measurement values, wherein the perception boundary comprises a plurality of color coordinates representing an outer boundary of accepted batches for the target standard sample color measurement value. Executing the predicted weight accuracy engine further includes determining a tinting confidence interval based upon the predicted color coordinate range and the perception boundary. The method further comprises displaying, on a display coupled to the color digitization processor, the perception boundary, the predicted color coordinate range, and the confidence interval. The method also includes providing, to the industrial mixing system, information relating to the predicted colorants and the masses thereof in response to an acceptance of the confidence interval, the predicted colorants and masses thereof for use in mixing, by the industrial mixing system, one or more colorants into the liquid batch. In another aspect, a liquid tinting system comprises a mixing system for mixing liquids a color digitization processor coupled to the mixing system, a color digitization database storing colorant information and product information associated therewith and coupled to the color digitization processor, and a memory storing computer-executable instructions. The instructions, when executed by the color digitization processor, configure the color tinting system for receiving, the liquid color information associated with a liquid batch, identifying a product identifier based on the liquid color information and the product information, and executing an artificial intelligence (AI) color prediction engine to predict colorants. Executing the Al color prediction engine comprises modeling, by one or more models, tinting of the liquid sample by predicting one or more colorants and colorant masses based on the liquid color information, the product identifier, and the colorant information. Executing the Al color prediction engine also comprises generating a model selection policy for the product identifier based on the plurality of historical batch sample color information and product information and selecting a preferred model of the one or more models based on the model selection policy for the product identifier. The liquid tinting system further comprises transmitting the colorants and colorant masses generated by the preferred model to the mixing system.

In yet another aspect, a method of tinting liquids in an industrial mixing system comprises collecting a liquid batch sample from a liquid batch in an industrial mixing system and measuring the liquid batch sample with a sensor to generate a batch color measurement value associated with the liquid batch sample. The method also comprises transmitting the batch color measurement value to a color digitization processor and executing, by the color digitization processor, an artificial intelligence (AI) tinting prediction engine. Executing the Al tinting prediction engine comprises storing the batch color measurement value in a color digitization database, the color digitization database comprising a plurality of historical batch sample color information, a plurality of target standard sample color measurement values, and a plurality of colorant information wherein the colorant information includes a colorant value. Executing the Al tinting prediction engine also comprises modeling, by one or more models, tinting of the liquid batch by weighting one or more of the delta errors, the batch color measurement value, a batch volume, the plurality of historical batch sample color information, or the plurality of colorant information to generate one or more predicted colorants and masses thereof from the plurality of colorant information to achieve the target standard sample color measurement. Executing the Al tinting prediction engine further comprises generating a model selection policy based on the plurality of historical batch sample color information and the target standard sample color measurement and selecting preferred predicted colorants and masses thereof by applying the model selection policy to the predicted colorants and masses thereof. The method further comprises receiving, by a color digitization processor, a batch color measurement value associated with a liquid batch in an industrial mixing system.

Other objects and features of the present invention will be in part apparent and in part pointed out herein.

### BRIEF DESCRIPTION OF THE DRAWINGS

FIG. 1 illustrates an artificial intelligence (AI) based tinting assistant system according to an embodiment.
FIG. 2A illustrates a sample display generated by the insights processor showing tinting iterations required by product cluster where the clusters are created based on colors.
FIG. 2B illustrates an embodiment of software enabling selection of colorants to generate a prediction of the tinting weights of the colorants.
FIG. 3 illustrates a process for predicting colorants to tint using an artificial intelligence (AI) tinting prediction engine.
FIG. 4 illustrates an embodiment of the AI tinting prediction engine for use in the process of FIG. 3.
FIG. 5 illustrates a method for digitizing tinting process information and training an AI tinting prediction engine.
FIG. 6A illustrates an isometric view of a color measurement unit.
FIG. 6B illustrates the interior of the color measurement unit during measurement of a liquid batch.
FIG. 7 illustrates using a virtual sensor and AI confidence engine to determine a confidence interval for mixing predicted colorants into a liquid batch according to an embodiment.
FIG. 8A illustrates a graph of a measured CIELAB coordinate of a batch and the predicted measurement after a tinting process within the perception boundary.
FIG. 8B illustrates a graph of measure coordinates for a set of batches, predicted results of mixing colorants, and the tolerance for a mixed batch compared to the perception boundary.

Corresponding reference characters indicate corresponding parts throughout the drawings.

### DETAILED DESCRIPTION

The features and other details of the concepts, systems, and techniques sought to be protected herein will now be more particularly described. It will be understood that any specific embodiments described herein are shown by way of illustration and not as limitations of the disclosure and the concepts described herein. Features of the subject matter described herein can be employed in various embodiments without departing from the scope of the concepts sought to be protected.

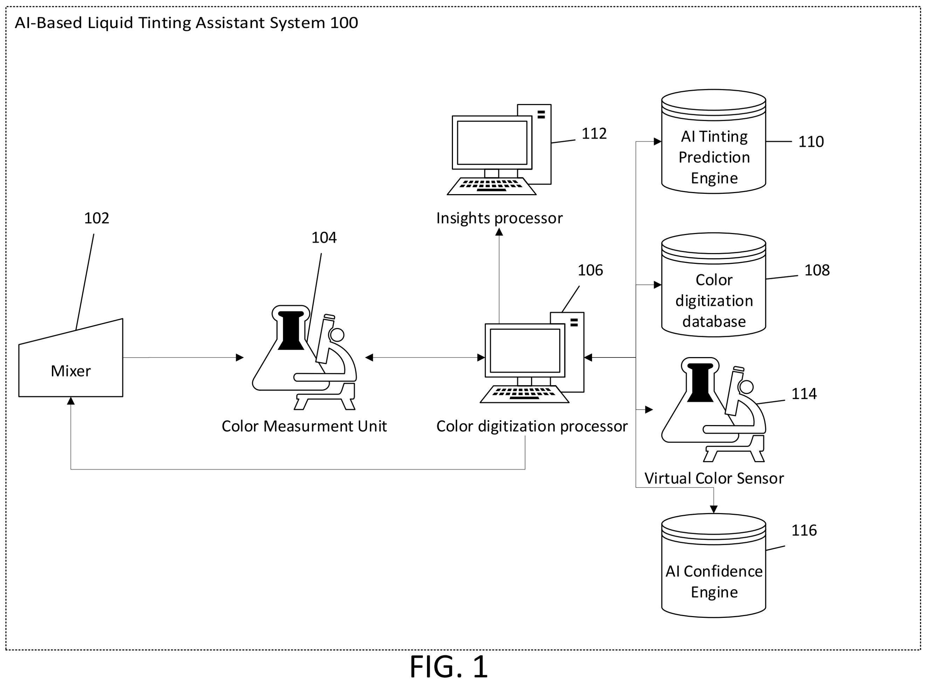

Referring to the figures and description below, a system 100 to tint liquids based on an artificial intelligence (Al), such as machine learning (ML), is disclosed. FIG. 1 is a block diagram illustrating the system 100 performing an example process embodying aspects of the disclosure. In one embodiment, an industrial mixing system 102 contains a liquid product to be tinted, such as a paint. In other embodiments, other liquid products may be tinted using a colorant. For example, example a colorant may be used in tinting food, beverage, or pharmaceutical products to achieve a desired color. For example, colorants may be used a process of tinting chocolate in industrial food production. In some embodiments the liquid product starts colored or tinted. A liquid batch sample is collected from the industrial mixing system 102 and transferred to a color measurement unit 104. The color measurement unit 104, further described below, measures the liquid batch sample to determine a color measurement value for the batch sample. As described above, a conventional tinting process requires using the liquid batch sample to create a batch test panel for comparison with a target reference panel rather than an evaluation of the liquid product itself. Aspects of the present disclosure increase the efficiency for production as it overcomes the need to repeatedly make test panels, thus, eliminating the time needed to create and cure multiple test panels. Similarly, batch production of food products such as chocolate can be improved by removing the cure time for chocolate. Further, the liquid color measurement system 100 reduces production costs for a batch because the system does not require the creation of physical batch test panels or product sample panels.

The color digitization processor 106 receives the liquid batch measurement and coordinates the tinting process. In some embodiments, the color digitization processor 106 couples with a display for displaying graphical information relating to the tinting process as well as input devices for configuring or updating the tinting process. In an embodiment, an operator of the tinting process may perform actions through interactive software, such as monitoring the tinting process, modifying aspects of the process reviewing the current tinting process, or reviewing historical tinting processes. The interactive software may be standalone or a component of the tinting process management software. The color digitization processor 106 connects with both a color digitization database 108 and an Al tinting prediction engine 110. In some embodiments, the Al tinting prediction engine 110 is executed by the color digitization processor 106.

The color digitization database 108 stores both information on the current tinting process, historical tinting processes, stock keeping unit (SKU) information for the target standard liquid samples and colorants added to tint the liquids. In some embodiments, the SKU information includes a SKU identifier, a SKU end application, color measurement values for the SKU, measurement dates, and an acceptable delta error. Historical batch tinting information can include information about the number of tinting operations, colorants required, colorant mass, colorant mixing times, batch sizes, batch dates, whether the batch was accepted and/or color measurement values for each product identifier, as well as information related to mapping colors to product identifiers. Colorant information stored within the color digitization database 108 includes one or more of colorant identifiers, and a strength measurement value.

In an embodiment, AI tinting prediction engine 110 comprises computer-executable instructions executed by the color digitization processor 106. The Al tinting prediction engine 110, as will be described in further detail below, receives the color measurement value, compares it to a corresponding value of a target color, predicts which colorants, and their masses, to be added to achieve the desired result matching the target color. The target color may be determined by a selected input color or automatically generated within the Al tinting prediction engine 110 based on the color values of the earlier accepted batches of the product.

In one or more embodiments, the AI tinting prediction engine 110 implements multiple models of tinting. For example, the Al tinting prediction engine 110 generates predictions through each of a linear model, a Gini Index based model, a near match model, and a smart tinting model. Then the Al tinting prediction engine 110, selects one or more preferred models of the tinting process based on a model selection policy. In some embodiments, the Al tinting prediction engine 110 generates a model selection policy for each SKU based on historical batch tinting data for that SKU. In other embodiments, an operator creates the model selection policy.

The color digitization processor 106 transmits the prediction information to an insights processor 112. In some embodiments, the insights processor 112 operates as a component within the color digitization processor 106. In other embodiments, the insights processor 112 operates standalone connected by network. In some embodiments, the insights processor 112 is connected to the color digitization processor 106 through a wireless network such as Wi-Fi or Bluetooth. The insights processor 112 executes instructions for transmitting the prediction information to an external display.

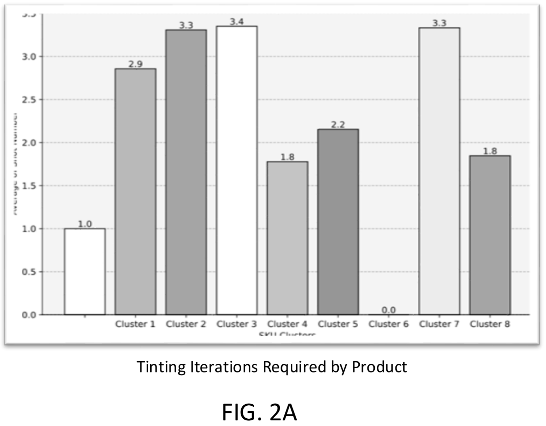

The insights processor 112 allows a plant manager or others supervising the tinting process access to details about the tinting process, including expected processing time and a predicted and/or actual number of iterations required to complete the tinting process. The insights processor 112 provides a plant manager or any other authorized plant personnel with real-time updates on the tinting process. Advantageously, up-to-date tinting process information enables an operator to accurately plan based on tinting process execution time thereby increasing plant efficiency. Further, the insights processor 112 in combination with the interactive software analyze the historical tinting information to display to an operator. The insights processor 112 evaluates the historical tinting information to evaluate products and provide feedback for the types of products requiring more iterations of tinting. For example, FIG. 2A illustrates a display showing tinting iterations required by product cluster according to an embodiment. The insights processor 112 and interactive software provide insights on the tinting process by clustering information from historical tinting processes to inform future tinting process planning.

The virtual color sensor 114 enables the color digitization processor 106 to predict the result of mixing predicted colorants into the liquid batch. In some embodiments, the virtual color sensor 114 is executed by the color digitization processor 106. The virtual color sensor 114, as will be described in further detail below, generates a predicted resulting color from mixing in either a predicted or input set of colorants and masses. In some embodiments, the predicted resulting color corresponds to an area of CIELAB color space. In other embodiments, the predicted resulting color corresponds to an area on other defined color spaces such as CIE XYZ, CIE L*C*h*, or CIELUV.

In an embodiment, AI confidence engine 116 comprises computer-executable instructions executed by the color digitization processor 106. The Al confidence engine 116, as will be described in further detail below, generates a perception boundary for each SKU within the color digitization database 108. Further, the Al confidence engine 116 determines a confidence interval for a predicted set of colorants based on the predicted result of mixing the colorants generated by the virtual color sensor 114 and the perception boundary for the target standard sample color. In some embodiments, the Al confidence engine 116 transmits the confidence interval to the insights processor 112 to display to the plant manager or others supervising the tinting process.

The insights processor 112 allows a plant manager or others supervising the tinting process access to details about the tinting process, including expected processing time and a predicted and/or actual number of iterations required to complete the tinting process. The insights processor 112 provides a plant manager or any other authorized plant personnel with real-time updates on the tinting process. Advantageously, up-to-date tinting process information enables an operator to accurately plan based on tinting process execution time thereby increasing plant efficiency. Further, the insights processor 112 in combination with the interactive software analyze the historical tinting information to display to an operator. The insight processor 112 evaluates the historical tinting information to evaluate products and provide feedback for the types of products requiring more iterations of tinting. For example, FIG. 2A illustrates a display showing tinting iterations required by product cluster according to an embodiment. The insights processor 112 and interactive software provide insights on the tinting process by clustering information from historical tinting processes to inform future tinting process planning.

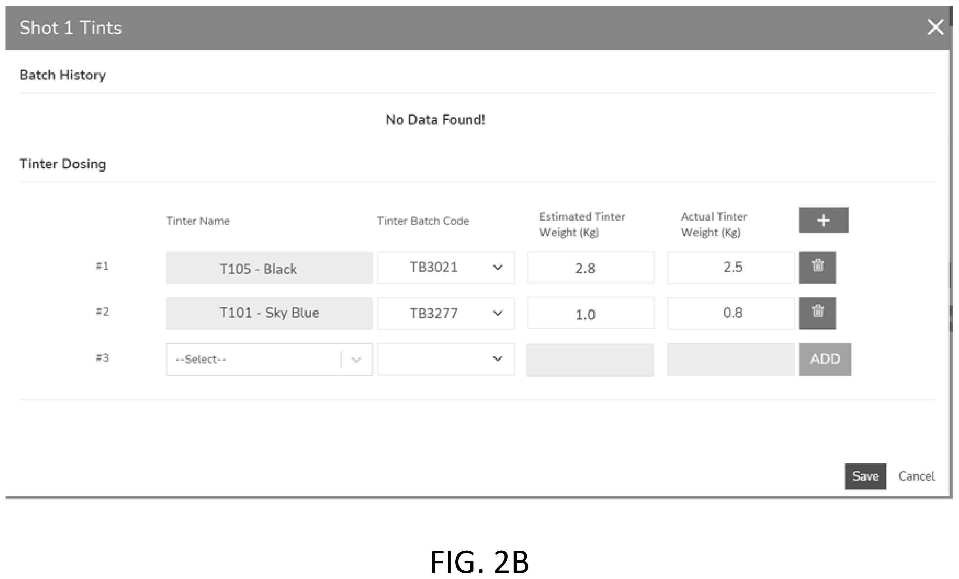

In some embodiments, the interactive software enables an operator to generate predictions for tinting process outcomes. As illustrated by FIG. 2B, in one embodiment, an operator inputs selected colorants and a target standard sample color to generate the prediction. Then the Al tinting prediction engine 110 predicts the mass of colorants to be added to achieve the desired target standard sample. Next, the operator performs tinting through the industrial mixing system 102 according to the prediction or the operator may override the prediction to alter the colorant masses. If the operator overrides the prediction, the changed values are transmitted to the color digitization database 108 to incorporate in retraining the Al tinting prediction engine 110. In another embodiment, a user inputs colorant data into the interactive software. Then, after selecting the colorants and mass of colorants to be used, the Al tinting prediction engine 110 using the virtual color sensor 114, generates a prediction of the delta effect on a liquid batch. The operator can adjust colorants added and the mass of colorants to find a desired outcome of the tinting process.

In parallel or serially with transmission to the insights processor 112, the color digitization processor 106 also transmits the predicted colorants and mass of colorants to the industrial mixing system 102 to complete the tinting process. The industrial mixing system 102 then adds the required colorants to the mixer to update the liquid batch. The resultant liquid batch may be processed multiple times to achieve the desired color by collecting subsequent measurement values.

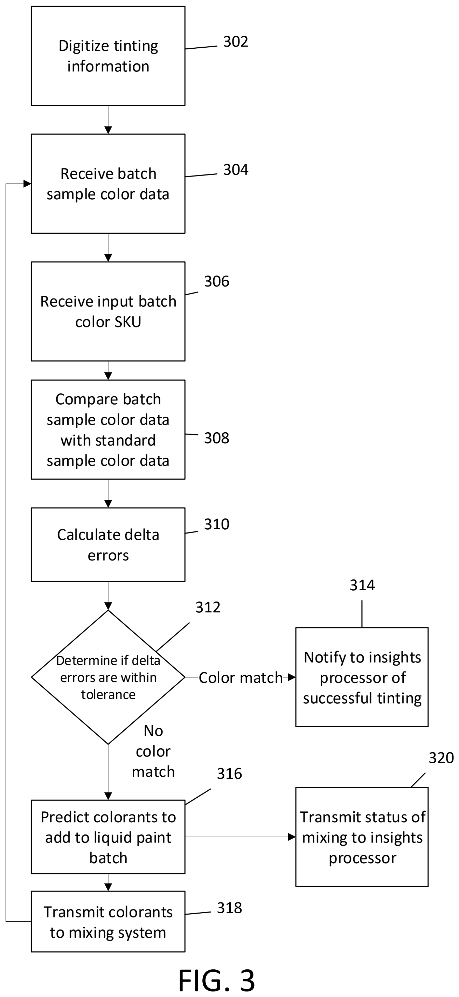

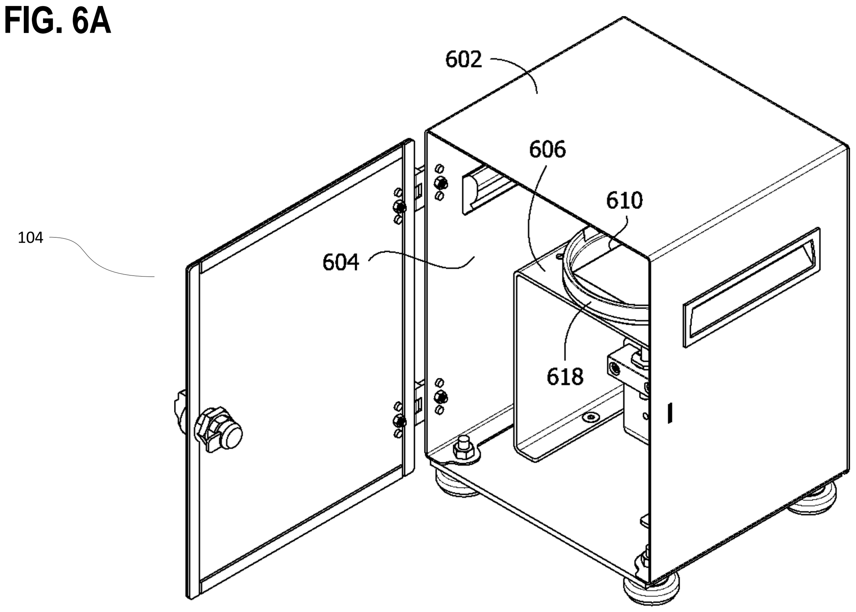

Referring now to FIG. 3, an example tinting process embodying aspects of the present disclosure is shown. The process begins with digitizing the tinting information at 302. Digitizing the batch information includes storing SKU liquid sample measurement values, colorant liquid sample measurement values, and previous batch sample measurement values in the color digitization database 108. The SKU liquid sample measurement values correspond to the potential target values for the tinting process. The process continues with receiving a measurement value of the batch sample color at 304. In some embodiments, the batch sample color is determined by a color measurement unit as seen in FIGS. 6A. In some embodiments, the color measurement value received by the color digitization processor 106 comprises a measurement in any of a variety of color spaces such as CIE XYZ, CIE L*a*b*, CIE L*C*h*, or CIELUV. After receiving the color measurement value, the operator inputs a product identifier or stock keeping unit (SKU) for the batch color at 306.

The tinting process continues at 308 by comparing the batch sample color measurement value with a target standard sample measurement value for the target color. In some embodiments the target standard sample color represents a color measurement value for an accepted liquid paint batch. In other embodiments, the target standard sample color corresponds to a color measurement value for an accepted batch of food or beverage product. In some embodiments, comparing the batch sample color measurement value with the target standard sample measurement value comprises calculating in the difference for each coordinate in a color space. For example, calculating the difference between each of the L*, a*, and b* coordinates in the CIELAB color space. In some embodiments, after calculating the difference for each coordinate, the color digitization processor 106 or Al tinting prediction engine 110 determines one or more delta error(s) at step 310. In one embodiment, a delta error in the CIELAB color space is determined by calculating the root mean square of all the coordinate differences. However, other methods of determining delta error(s) can be applied. The Al prediction engine 110 then evaluates a status based on the delta error(s) at step 312. In one embodiment, the Al prediction engine 110 predicts an acceptable tolerance of delta error(s) for the target standard sample value based on historical batch information including whether a given batch for the same SKU was accepted or rejected. If the delta error(s) are within an acceptable tolerance for the target standard sample value, the tinting process is complete. The color digitization processor 106 then transmits this status to the insights processor 112. In some embodiments, the color measurement unit captures another color measurement value for the resultant batch to update the color digitization database with further information.

Table I, below shows examples of batch information and calculating delta error within the CIELAB color space:

**TABLE I**

| | | | Liquid Sample Measurement | | | Standard Sample Measurement | | | Error | | | |
|---|---|---|---|---|---|---|---|---|---|---|---|---|
| Batch ID | SKU ID | Date | L | A | B | L | A | B | ΔL | ΔA | ΔB | ΔE |
| B1095 | S90H88 | 8/16/2024 | 83.72 | 0.72 | -1.07 | 84.02 | 0.71 | -0.86 | 0.30 | -0.01 | 0.21 | 0.37 |
| B1094 | S33FF6 | 8/16/2024 | 47.09 | 4.21 | -19.92 | 46.75 | 3.92 | -20.54 | -0.34 | -0.29 | -0.62 | 0.76 |
| B1093 | S79GD1 | 8/16/2024 | 78.82 | -0.55 | 8.75 | 78.77 | -0.47 | 8.75 | -0.05 | 0.08 | 0.00 | 0.09 |

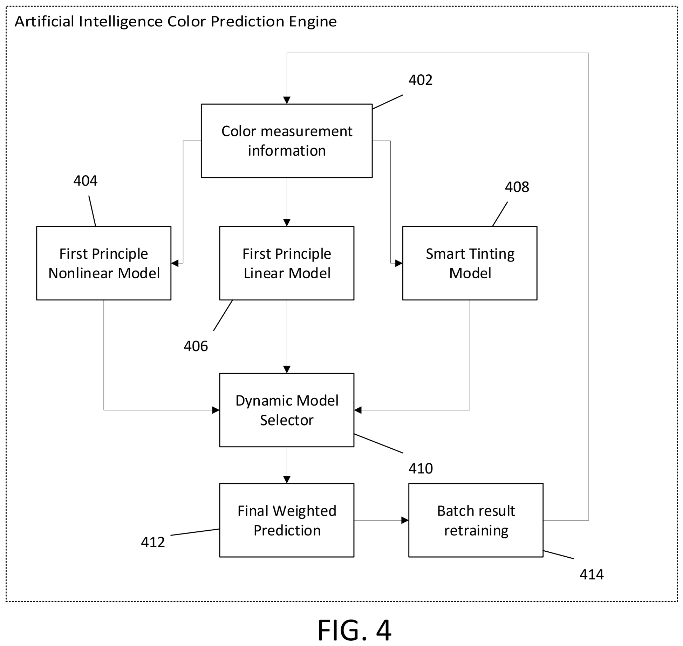

If the delta error(s) are not within an acceptable tolerance, the tinting process continues with further tinting. At step 316, the Al tinting prediction engine predicts colorants and mass of colorants needed to obtain the target standard sample measurement value by modeling the tinting process. This process is illustrated in FIG. 4, which is further described below. After predicting colorants to be added to the liquid batch, the color digitization processor 106 transmits the colorants and needed mass to the industrial mixing system 102 at step 318. In some embodiments, an operator may override the predicted mass of colorants. For example, an operator selects masses at 70% of the prediction in order to test the batch after the next iteration of mixing. The mixing system then proceeds with tinting the liquid batch with the colorants with the predicted mass or the operator input mass. After tinting the liquid, the color digitization processor 106 transmits the predicted colorants to the insights processor 112 along with information regarding expected tinting operations required and/or time to complete the tinting process at step 320. Then, after the mixing is completed by the industrial mixing system 102, the tinting may restart at step 304 by collecting a second batch sample from the resultant batch.

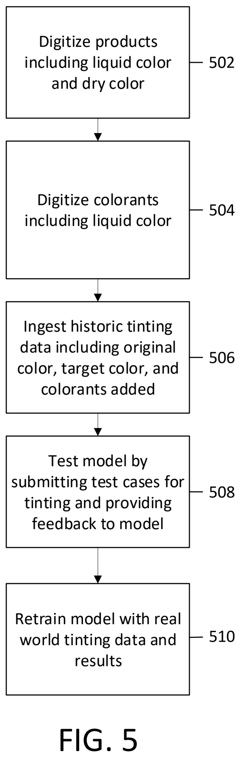

Referring now to FIG. 4, the AI tinting prediction engine 110 comprises one or more models of the tinting process generating one or more predictions for tinting. The Al tinting prediction engine 110 begins by receiving the color measurement information 402. In some embodiments, the color measurement information received from the color measurement unit 104. In other embodiments, a color sensor generates the color measurement information. In one embodiment, color measurement information 402 comprises a color measurement value, such as a CIELAB coordinate, a calculation of delta error(s), a batch volume, and target color information which comprises a target color measurement value. Then one or more models weight the color information to predict which colorants and needed mass of each to add to the liquid batch to achieve a match to the target color after application and drying. FIG. 4 illustrates one embodiment where the Al prediction engine 110 feeds the color information into multiple models. Prediction generating models include any of various machine learning models. The present embodiment of FIG. 4 illustrates using a linear model 406, a nonlinear model (e.g. a Gini Index based model or a near match model), and a smart tinting model 408. While several examples models are illustrated, those of skill in the art will appreciate that multiple machine learning models are suitable based on the application and implementation. The smart tinting model 408 is a machine learning model which has been trained based on historical tinting information and colorant information as described below, see FIG. 5.

Each model generates a prediction of weights for tinting. As each model receives the color measurement information 402, it generates a prediction. Each model may be separately trained and set different weights for each property under consideration depending on the model. In one embodiment, the models are weighted based on a strength factor. The measurement of color in the digital space may not accurately represent a color representation in the physical space. As such, one or more of the prediction models may be given a weighting to update colors after the initial prediction to better reflect physical space color. The prediction from each model is then fed into a dynamic model selector 410.

The dynamic model selector 410 generates a final weighted prediction 412 for the tinting operation. The dynamic model selector 410 generates a model selection policy for each SKU based on historical batch information and which models previously were most successful at obtaining the target standard sample color for the SKU. In some embodiments, the dynamic model selector 410 selects a single prediction of the input models based on the model selection policy. Since the dynamic model selector 410 generates a model selection policy on the historical batch information, the dynamic model selector 410 continuously learns and updates from each batch operation. In some embodiments, the dynamic model selector 410 generates a composite weight based on the input models predictions. In still other embodiments, the dynamic model selector 410 acts as a second layer of Al prediction by generating weights for each model prediction to generate a single weight through a machine learning model.

After generation of the final weighted prediction 412, the batch result retrains the model 414. In some embodiments, the industrial mixing system 102 uses the final weighted prediction 412 to add colorants to a liquid batch. After, resultant generated color measurement information is fed back into the Al color prediction engine for retraining at 402. In testing the model, rather than feeding back in a new sample measured after the tinting process, the color measurement information 402 is a result from historical tinting data. Each of the models undergoes re-weighting of their various inputs including the dynamic model selector 412 in embodiments where the dynamic model selector 410 is a machine learning model itself. Aspects to the present disclosure improve consistency of the tinting process. Conventional models of tinting require an expert in the tinting process to evaluate batch sample panels and standard reference panels to predict one or more colorants needed for the liquid batch. As a result, the process can vary based on the knowledge of the expert and subjective observation. However, the process as presently disclosed, improves the process by ensuring consistent predictions for a given target color due to the digitizing the knowledge of a tinting expert and consistent, standardized modeling through the Al tinting prediction engine 110.

Referring now to FIG. 5, the AI tinting prediction engine 110 of FIG. 4 comprises a tinting model trained on historical tinting information, and FIG. 5 illustrates one embodiment of a method of digitizing tinting process information and training the Al tinting prediction engine 110. First at step 502, products are digitized within the color digitization database 108. Digitization of products includes storing information comprising a product identifier or SKU, a color measurement value for the liquid product, a color measurement value for the dry product, and previous tinting information. Previous tinting information further comprises a color measurement value for the liquid product at each step of the tinting process, colorants added for each tinting step, mass of colorants added, and any dry color measurement values for each step available.

Next at step 504, colorant information is digitized for use within the AI tinting prediction engine 110. Digitizing colorant information includes storing information comprising a colorant identifier, any manufacturing information if available, and a colorant strength which can be measured, predicted, or provided by the manufacturer. A colorant strength represents the effectiveness of a colorant at tinting a liquid product. A colorant having a high colorant strength requires less mass of the colorant added to the liquid batch to achieve the desired outcome, whereas a colorant having a low colorant strength requires more mass of the colorant to achieve the desired outcome. Colorant information also comprises historical information regarding use of the colorant, such as which products include use of the colorant and mass of colorant used. In some embodiments, the Al tinting prediction engine 110 predicts a colorant strength by weighting previous historical tinting process information based on the mass of colorant used.

The training of the model begins at step 506 by ingesting the historical tinting information. A curated set of the historical tinting information is synthesized for training the model. The information is selected based on various criteria depending on factors such as tinting outcomes, reliability of the information, and sufficiency of detailed process information for each step of the tinting process. Similarly, the colorant information is also curated to optimize training of the model. As a part of curating the training set of information, a curated set of testing information will also be generated. Then the model is trained based off the curated historical tinting information as well as the curated colorant information.

After initial training of the model, the model is tested at step 508. Testing the model involves submitting curated historical tinting process information from the test set to the model to determine a prediction. The prediction is then compared to the historical process result to determine the accuracy of the prediction. Then the weights for the colorant information or product information are updated based on the accuracy of the prediction. Finally, the model retrains further at step 510 as the tinting process generates new tinting information to feed back into the model. As a result, the model continually learns from the generated predicting colorants and their resultant color measurement values.

Referring now to FIG. 6A, the system for tinting liquids in some embodiments implements color measurement unit 104. The color measurement unit 104 provides accurate color measurement of liquids including highly viscous liquids. The color measurement unit 104 in the illustrated embodiment comprises a container 602 within an interior area 604 in which to measure the liquid. The container 602 may be of any opaque material to prevent outside light from interfering with the measurement. In some embodiments, color measurement unit 104 further comprises a hinged door which seals to the exterior of the container 602 to prevent all light from interior area 604. The color measurement unit 104 further comprises an interior area 604 within container 602 designed for testing a liquid batch sample. Within the interior area 604, a testing shelf 606 is mounted inside container 602. In the present embodiment, the testing shelf 606 includes a transparent testing surface 610 formed therein. In some embodiments, the testing shelf 606 only comprises the transparent testing surface 610. The transparent testing surface 610 may be made of a light permeable material, such as glass or plastic, enabling the testing of the liquid batch sample from below the testing shelf 606.

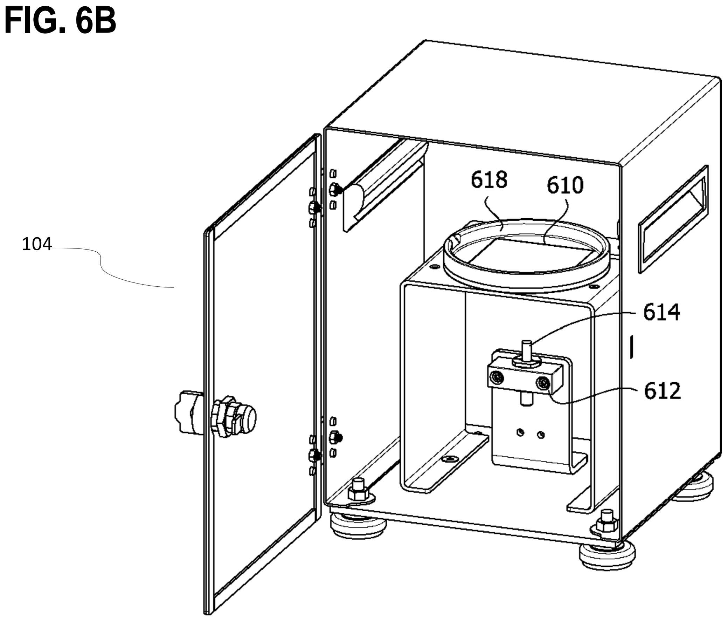

Referring now to FIG. 6B., beneath the testing shelf 606 coupled to the base of the container is a mount 612 for attaching a sensor 614. In some embodiments, the mount 612 is adjustable enabling the sensor 614 to be moved closer or further away from the transparent testing surface 610 for more accurate testing. In some embodiments the sensor 614 is a spectrophotometer. The spectrophotometer may be coupled with a controller or another device for transmitting the data captured by the spectrophotometer to the color digitization processor 106. An example of a suitable spectrophotometer is the Micro-Epsilon CFS2-M20-E-2400. Similarly, a suitable controller for transmitting the sensor data is the Micro-Epsilon colorSENSOR CFO200.

In some aspects, the position of the sensor 614 enables quick accurate testing of liquid samples. A batch sample within a testing container 618 is placed upon the transparent interior testing surface 610. Then the sensor 614 captures a color measurement value of the batch sample, which may be processed by a separate controller or the color digitization processor 106. As previously discussed, conventional methods of detecting color cause the spectrophotometer to be oriented above the test sample. According to the conventional arrangement, the meniscus of the liquid sample and/or air bubbles within a highly viscous liquid are causes of color measurement error. In the present embodiment, a sample can quickly be placed within the interior testing surface 610 and measured with the sensor 614 without significant reconfiguration to adjust for the meniscus of the liquid or delaying to allow air bubbles to escape the liquid. Moreover, color measurement unit 104 is capable of capturing color measurement values from a liquid sample utilizing the test configuration of FIGS. 6A and 6B thus avoiding the drying time associated with conventional color measurements.

As FIG. 1 illustrates, in some embodiments, the color measurement unit 104 of FIG. 6A and FIG. 6B is coupled to the color digitization processor 106. The direct connection between the color measurement unit 104 and the color digitization processor 106 enables direct management of the sensor 614 from the color digitization processor 106. Further, the direct link ensures safe rapid storage of the liquid batch measurement value without relying on an external network. In some embodiments, the color digitization processor 106 further comprises the insights processor 112 and the Al tinting prediction engine 110 as subcomponents of the tinting process software. In such an embodiment, the color digitization processor 106 operates the entirety of the tinting and prediction process and displays all information regarding the current tinting process and historical tinting processes. In some embodiments, the color measurement unit 104 and color digitization processor 106 are coupled by a wireless network, such as Wi-Fi or Bluetooth.

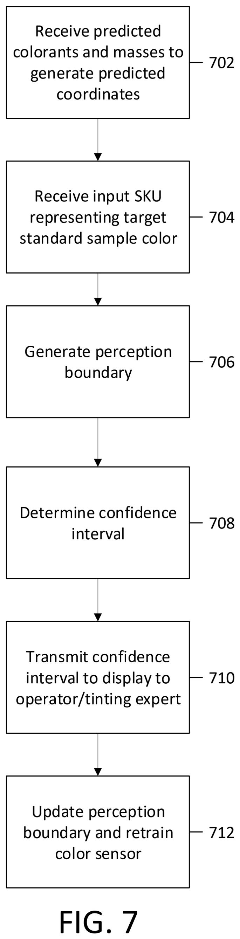

FIG. 7 is a flow diagram illustrating a process of determining a confidence interval for a final weighted prediction through an Al confidence engine. At 702, a final prediction of selected colorants and their respective masses is input into the virtual color sensor 114, see FIG. 1. The virtual color sensor 114 receives an input of the colorants and their weights to predict a resulting CIELAB coordinate for mixing the colorants into the batch. In some embodiments, the predicted CIELAB coordinate includes a range of potential output coordinates in the CIELAB color space. In some embodiments, the virtual color sensor 114 continuously updates based on subsequent batch information. The virtual color sensor 114 receives the results of mixing the predicted colorants into the batch and determines whether the prediction yielded the expected target standard sample color. The virtual color sensor 114 then updates the weights of the model based upon the accuracy of the prediction. Thus, the model continues to refine its predictions as more liquid batches are mixed.

Then AI confidence engine 116, see FIG. 1, receives an input SKU representing the target standard sample color at 704. Next at 706, the Al confidence engine 116 determines a perception boundary for the target standard sample color. The perception boundary represents the human perception of color for a given environment. Various factors affect color perception, and a tinting expert may accept a batch color mix as a match even when the CIELAB coordinate may not precisely match the target standard sample color. Such factors impacting perception include lighting conditions, variability in material type, and a tinting expert's personal perception. By generating the perception boundary, the Al confidence engine 116 accurately predicts whether the real world color resulting from a liquid batch would be accepted for the desired target sample color based on a tinting expert's visual perception of the liquid batch. Determining a perception boundary for the target standard sample color involves an Al model trained on historical tinting information. In some embodiments, the Al confidence engine 116 comprises a machine learning model. The Al confidence engine determines a perception boundary based on historical tinting information for the target standard sample color. Historical tinting information includes whether a given batch was accepted for the target standard sample color and the CIELAB coordinates of the resulting batch color. The model develops a three-dimensional boundary based on the area of CIELAB coordinates that were accepted for the target standard sample while accounting for outliers within the historical tinting information.

The AI confidence engine 116 determines a confidence interval for mixing operation based on the virtual color sensor's predicted CIELAB coordinate range and the perception boundary at step 708. The perception boundary creates an area of CIELAB color space where the Al confidence engine 116 expects that a tinting expert would accept the batch color result for the target standard sample color. By mapping the predicted colorant weights through the virtual sensor 114, the resulting CIELAB coordinate space can be evaluated relative to the perception boundary space. A higher overlap between the perception boundary space and the virtual color sensor 114 color space indicates a higher confidence that the resulting batch will match the target standard sample color. Because the virtual color sensor's 114 predicted coordinates and the perception boundary each represent a three-dimensional area, a confidence interval can be determined by measuring the percentage of the predicted coordinate area falling within the perception boundary.

In some embodiments, the resulting confidence interval is transmitted to the color digitization processor 106 at step 710. In some embodiments, the color digitization processor 106 displays the confidence interval along with a color representing the level of confidence. For example, the display of the color digitization processor 106 may show the batch tinting process in a green color when the confidence interval exceeds 80%, a yellow color when the confidence interval is between 60% and 80%, or a red color when the confidence interval falls below 60%. The color digitization processor 106 then may allow an operator to determine to proceed with the tinting process. Further, the operator may adjust the colorants based upon the confidence interval. In response to an adjustment by the operator, the Al confidence engine 116 receives the input colorants and weights to generate a new predicted result through the virtual color sensor 114. Thus, a second confidence interval based upon the operator input can be generated. In some embodiments, mixing of the liquid batch with the colorants proceeds if the confidence interval exceeds 80%. In other embodiments, an operator may accept the predicted colorants to proceed with mixing the liquid batch based on the confidence interval.

The AI confidence engine 116 updates the perception boundary for the SKU representing the target standard sample based on the subsequent tinting data at 712. After the liquid batch is eventually tinted with the predicted colorants and their masses, the Al confidence engine 116 receives information based on the results. In some embodiments, the information includes a resulting liquid batch measurement value received from a color measurement unit after performing the mix. This resulting measurement can be used to retrain the virtual color sensor 114. In further embodiments, the information includes whether the tinting expert accepted the resulting liquid batch as acceptable for the target standard sample. Based on whether the resulting liquid batch was accepted or rejected, the Al confidence engine 116 updates the perception boundary for the SKU using the resulting liquid batch measurement and/or the virtual color sensor's predicted CIELAB coordinates. Because both the Al confidence engine 116 and the virtual sensor retrain on the resulting measurement and the tinting expert's decision-making, both the Al confidence engine 116 and the virtual color sensor 114 develop on the tinting expert's knowledge and visual perception. As the both the Al confidence engine 116 and virtual color sensor 114 train, they both better replicate the expertise of the tinting expert.

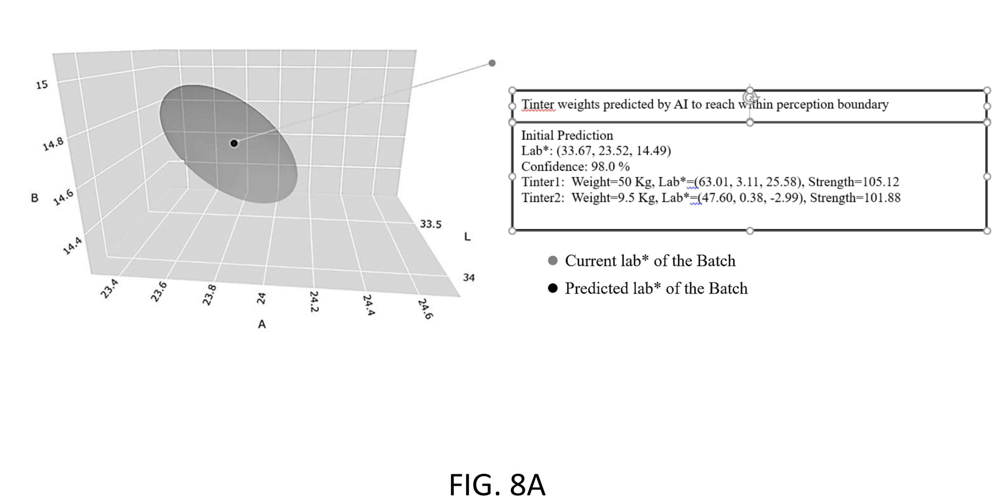

FIG. 8A illustrates a graph of a measured CIELAB coordinate of a batch and the predicted measurement after a tinting process within the perception boundary. As shown the measured batch has a CIELAB coordinate initially outside the boundary. The Al tinting prediction engine 110 predicts a set of colorants or tinters to add to the batch to achieve the desired target color. Then the virtual color sensor 114 predicts a resulting batch color, shown in the graph as coordinates (33.67, 23.52, 14.49). Finally, the Al confidence engine 116 compares the predicted resulting batch to the perception boundary and calculates a confidence, shown here as 98%.

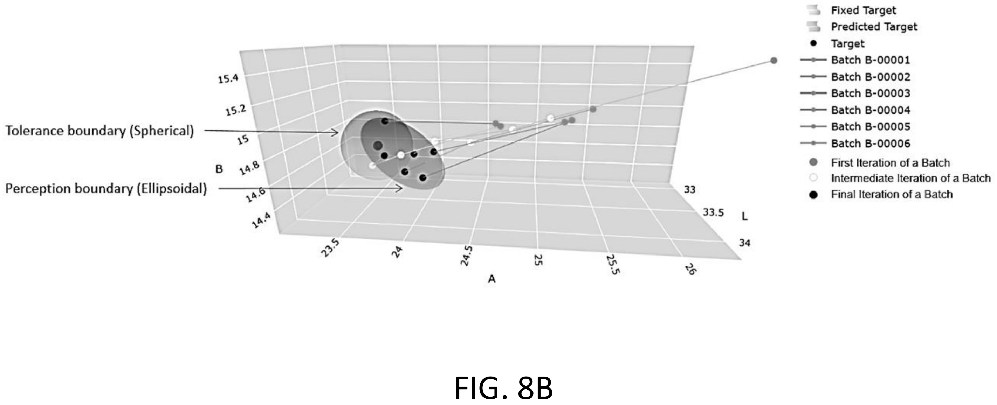

FIG. 8B illustrates a graph of measure coordinates for a set of batches, predicted results of mixing colorants, and the tolerance for a mixed batch compared to the perception boundary. As show several batches for a target sample are graphed, ex. Batch B-00001, B-000002, etc. Predicted results of mixing colorants for two iterations are shown with an intermediate iteration and a final iteration. Thus the final iterations each have a tolerance boundary based on the predicted resulting batch generated by the virtual color sensor 114. This tolerance may be compared to the perception boundary generated by the Al confidence engine 116 to determine a confidence interval.

Commonly assigned Indian Patent Application No. 202411097083, filed December 9, 2024, the entire disclosure of which is incorporated by reference, discloses an artificial intelligence (AI) based batch tinting assistant. A color measurement unit receives a liquid batch sample and measures the sample to generate a color measurement value. A color digitization processor receives the measurement and executes an Al tinting prediction engine to define delta errors and predict colorants and masses to add to the liquid batch to obtain a target standard sample. The predicted colorants are then transmitted to the mixing system and mixed into the liquid batch.

Embodiments of the present disclosure may comprise a special purpose computer including a variety of computer hardware, as described in greater detail herein.

For purposes of illustration, programs and other executable program components may be shown as discrete blocks. It is recognized, however, that such programs and components reside at various times in different storage components of a computing device, and are executed by a data processor(s) of the device.

Although described in connection with an example computing system environment, embodiments of the aspects of the invention are operational with other special purpose computing system environments or configurations. The computing system environment is not intended to suggest any limitation as to the scope of use or functionality of any aspect of the invention. Moreover, the computing system environment should not be interpreted as having any dependency or requirement relating to any one or combination of components illustrated in the example operating environment. Examples of computing systems, environments, and/or configurations that may be suitable for use with aspects of the invention include, but are not limited to, personal computers, server computers, hand-held or laptop devices, multiprocessor systems, microprocessor-based systems, set top boxes, programmable consumer electronics, mobile telephones, network PCs, minicomputers, mainframe computers, distributed computing environments that include any of the above systems or devices, and the like.

Embodiments of the aspects of the present disclosure may be described in the general context of data and/or processor-executable instructions, such as program modules, stored one or more tangible, non-transitory storage media and executed by one or more processors or other devices. Generally, program modules include, but are not limited to, routines, programs, objects, components, and data structures that perform particular tasks or implement particular abstract data types. Aspects of the present disclosure may also be practiced in distributed computing environments where tasks are performed by remote processing devices that are linked through a communications network. In a distributed computing environment, program modules may be located in both local and remote storage media including memory storage devices.

In operation, processors, computers and/or servers may execute the processor-executable instructions (e.g., software, firmware, and/or hardware) such as those illustrated herein to implement aspects of the invention.

Embodiments may be implemented with processor-executable instructions. The processor-executable instructions may be organized into one or more processor-executable components or modules on a tangible processor readable storage medium. Also, embodiments may be implemented with any number and organization of such components or modules. For example, aspects of the present disclosure are not limited to the specific processor-executable instructions or the specific components or modules illustrated in the figures and described herein. Other embodiments may include different processor-executable instructions or components having more or less functionality than illustrated and described herein.

The order of execution or performance of the operations in accordance with aspects of the present disclosure illustrated and described herein is not essential, unless otherwise specified. That is, the operations may be performed in any order, unless otherwise specified, and embodiments may include additional or fewer operations than those disclosed herein. For example, it is contemplated that executing or performing a particular operation before, contemporaneously with, or after another operation is within the scope of the invention.

When introducing elements of the invention or embodiments thereof, the articles "a," "an," "the," and "said" are intended to mean that there are one or more of the elements. The terms "comprising," "including," and "having" are intended to be inclusive and mean that there may be additional elements other than the listed elements.

Not all of the depicted components illustrated or described may be required. In addition, some implementations and embodiments may include additional components. Variations in the arrangement and type of the components may be made without departing from the scope of the claims as set forth herein. Additional, different or fewer components may be provided and components may be combined. Alternatively, or in addition, a component may be implemented by several components.

The above description illustrates embodiments by way of example and not by way of limitation. This description enables one skilled in the art to make and use aspects of the invention, and describes several embodiments, adaptations, variations, alternatives and uses of the aspects of the invention, including what is presently believed to be the best mode of carrying out the aspects of the invention. Additionally, it is to be understood that the aspects of the invention are not limited in its application to the details of construction and the arrangement of components set forth in the following description or illustrated in the drawings. The aspects of the invention are capable of other embodiments and of being practiced or carried out in various ways. Also, it will be understood that the phraseology and terminology used herein is for the purpose of description and should not be regarded as limiting.

It will be apparent that modifications and variations are possible without departing from the scope of the invention defined in the appended claims. As various changes could be made in the above constructions and methods without departing from the scope of the invention, it is intended that all matter contained in the above description and shown in the accompanying drawings shall be interpreted as illustrative and not in a limiting sense.

In view of the above, it will be seen that several advantages of the aspects of the invention are achieved and other advantageous results attained.

The Abstract and Summary are provided to help the reader quickly ascertain the nature of the technical disclosure. They are submitted with the understanding that they will not be used to interpret or limit the scope or meaning of the claims. The Summary is provided to introduce a selection of concepts in simplified form that are further described in the Detailed Description. The Summary is not intended to identify key features or essential features of the claimed subject matter, nor is it intended to be used as an aid in determining the claimed subject matter.

Further aspects of the invention are provided by the subject matter of the following clauses:
Clause 1. A method of controlling a tinting of liquids in an industrial mixing system, the method comprising: receiving, at a color digitization processor, a batch color measurement value associated with a liquid batch in an industrial mixing system; executing, by the color digitization processor, an artificial intelligence (AI) tinting prediction engine, wherein executing the Al tinting prediction engine comprises: storing the batch color measurement value in a color digitization database, the color digitization database comprising a plurality of historical batch sample color information, a plurality of target standard sample color measurement values, and a plurality of colorant information, wherein the colorant information includes a colorant value; and modeling, by one or more models, tinting of the liquid batch by weighting one or more of the batch color measurement value, a batch volume, the plurality of historical batch sample color information, or the plurality of colorant information to generate one or more predicted colorants and masses thereof from the plurality of colorant information to achieve the target standard sample color measurement; executing, by the color digitization processor, a virtual color sensor, wherein executing the virtual color sensor comprises: performing virtual tinting based upon the batch color measurement value, the predicted colorants, and the masses thereof; and generating a predicted color coordinate range based upon the virtual tinting; executing, by the color digitization processor, a predicted weight accuracy engine, wherein executing the predicted weight accuracy engine comprises: generating a perception boundary based on the plurality of historical batch sample color information and the plurality of target standard sample color measurement values, wherein the perception boundary comprises a plurality of color coordinates representing an outer boundary of accepted batches for the target standard sample color measurement value; and determining a tinting confidence interval based upon the predicted color coordinate range and the perception boundary; displaying, on a display coupled to the color digitization processor, the perception boundary, the predicted color coordinate range, and the confidence interval; and providing, to the industrial mixing system, information relating to the predicted colorants and the masses thereof in response to an acceptance of the confidence interval, the predicted colorants and masses thereof for use in mixing, by the industrial mixing system, one or more colorants into the liquid batch.
Clause 2. The method of Clause 1, wherein the one or more models comprise at least one of a linear model, a nonlinear model, a smart tinting model, a Gini index based model, or a near match model.
Clause 3. The method of Clause 1 or Clause 2, wherein executing the AI tinting prediction engine further comprises: generating a model selection policy for each of the plurality of the target standard sample color measurement values based on the plurality of historical batch sample color information; and selecting a preferred model based on the model selection policy for the target standard sample color measurement value.
Clause 4. The method of any of Clauses 1 to 3, wherein the method further comprises: collecting, after mixing the one or more colorants, a resultant liquid sample from the industrial mixing system and a batch acceptance status; analyzing the resultant liquid sample by the sensor to generate a resultant color measurement value associated with the resultant liquid sample; and transmitting the resultant color measurement value and the batch acceptance status to the color digitization processor.
Clause 5. The method of Clause 4, wherein executing the predicted weight accuracy engine further comprises: re-generating the perception boundary based on the plurality of historical batch sample color information, the plurality of target standard sample color measurement values, the resultant color measurement value, and the batch acceptance status.
Clause 6. The method of any of Clauses 1 to 5, wherein the method further comprises: sending the confidence interval to an insights processor wherein the insights processor is configured to transmit graphical data of the predicted color coordinate range, the perception boundary, and the confidence interval to a display.
Clause 7. The method of Clause 6, wherein the method further comprises: receiving, after sending the confidence interval to the insights processor, an input of colorants and weights thereof; re-executing, by the color digitization processor, the virtual color sensor, wherein re-executing the virtual color sensor comprises: performing virtual tinting based upon the batch color measurement value, the input colorants, and the masses thereof; and generating an updated predicted color coordinate range based upon the virtual tinting; and re-executing, by the color digitization processor, the predicted weight accuracy engine, wherein re-executing the predicted weight accuracy engine comprises: determining a tinting confidence interval based upon the updated predicted color coordinate range and the perception boundary.
Clause 8. The method of any of Clauses 1 to 7, wherein the predicted color coordinate range comprises a CIELAB coordinate range.
Clause 9. The method of any of Clauses 1 to 8, wherein the perception boundary comprises a CIELAB coordinate range.
Clause 10. The method of any of Clauses 1 to 9, wherein the liquid batch comprises at least one of a paint batch, a food product batch, or a beverage product batch.
Clause 11. A liquid tinting system comprising: a mixing system for mixing liquids; a color digitization processor coupled to the mixing system; a color digitization database coupled to the color digitization processor, the color digitization database storing colorant information and product information associated therewith; and a memory storing computer-executable instructions that, when executed by the color digitization processor, configure the color tinting system for: receiving liquid color information associated with a liquid batch; identifying a product identifier based on the liquid color information and the product information; and executing an artificial intelligence (AI) color prediction engine to predict colorants, wherein executing the Al color prediction engine comprises: modeling, by one or more models, tinting of the liquid sample by predicting one or more colorants and colorant masses based on the liquid color information, the product identifier, and the colorant information; generating a model selection policy for the product identifier based on the plurality of historical batch sample color information and product information; selecting a preferred model of the one or more models based on the model selection policy for the product identifier; and transmitting the colorants and colorant masses generated by the preferred model to the mixing system.
Clause 12. The system of Clause 11, wherein the one or more models comprise at least one of a linear model, a nonlinear model, a smart tinting model, a Gini index based model, or a near match model.
Clause 13. The system of Clause 11 or Clause 12, wherein the preferred model comprises a combination of a first model of the one or more models and a second model of the one or more models.
Clause 14. The system of any of Clauses 11 to 13, wherein the memory stores computer-executable instructions that, when executed by the color digitization processor, further configure the color tinting system, after selecting a preferred model, for: executing a virtual color sensor, wherein executing the virtual color sensor comprises: performing virtual tinting based upon the liquid color information, the colorants and colorant masses generated by the preferred model; and generating a predicted color coordinate range based upon the virtual tinting; executing, by the color digitization processor, a predicted weight accuracy engine, wherein executing the predicted weight accuracy engine comprises: generating a perception boundary based on liquid color information and the product information, wherein the perception boundary comprises a plurality of color coordinates representing an outer boundary of accepted batches for the target standard sample color measurement value; and determining a tinting confidence interval based upon the predicted color coordinate range and the perception boundary; and displaying, on a display coupled to the color digitization processor, the perception boundary, the predicted color coordinate range, and the confidence interval.
Clause 15. The system of Clause 14, wherein the memory stores computer-executable instructions that, when executed by the color digitization processor, further configure the color tinting system, after executing the predicted weight accuracy engine, for: sending the confidence interval to an insights processor wherein the insights processor is configured to transmit graphical data of the predicted color coordinate range, the perception boundary, and the confidence interval to a display.
Clause 16. The system of Clause 14 or Clause 15, wherein the predicted color coordinate range comprises a CIELAB coordinate range.
Clause 17. The system of any of Clauses 11 to 16, wherein the memory stores computer-executable instructions that, when executed by the color digitization processor, further configure the color tinting system for: receiving a result of mixing the colorants and colorant masses; and re-executing the artificial intelligence (AI) color prediction engine to update the model selection policy, wherein re-executing the Al color prediction engine comprises: updating the model selection policy for the product identifier based on the result of mixing the colorants and colorant masses, the plurality of historical batch sample color information, and product information.
Clause 18. A method of tinting liquids in an industrial mixing system, the method comprising: receiving, by a color digitization processor, a batch color measurement value associated with a liquid batch in an industrial mixing system; executing, by the color digitization processor, an artificial intelligence (AI) tinting prediction engine, wherein executing the Al tinting prediction engine comprises: storing the batch color measurement value in a color digitization database, the color digitization database comprising a plurality of historical batch sample color information, a plurality of target standard sample color measurement values, and a plurality of colorant information wherein the colorant information includes a colorant value; and modeling, by one or more models, tinting of the liquid batch by weighting one or more of the delta errors, the batch color measurement value, a batch volume, the plurality of historical batch sample color information, or the plurality of colorant information to generate one or more predicted colorants and masses thereof from the plurality of colorant information to achieve the target standard sample color measurement; generating a model selection policy based on the plurality of historical batch sample color information and the target standard sample color measurement; and selecting preferred predicted colorants and masses thereof by applying the model selection policy to the predicted colorants and masses thereof; receiving, by the industrial mixing system, the preferred predicted colorants and the masses thereof; and mixing, by the industrial mixing system, one or more colorants into the liquid batch based on the preferred predicted colorants and the masses thereof.
Clause 19. The method of Clause 18, wherein the one or more models comprise at least one of a linear model, a nonlinear model, a smart tinting model, a Gini index based model, or a near match model.
Clause 20. The method of Clause 18 or Clause 19, wherein applying the model selection policy to the predicted colorants and masses thereof further comprises: applying the model selection policy to the predicted colorants and masses thereof by combining a first predicted colorants and masses thereof of the one or more predicted colorants and the masses thereof resulting from a first model of the one or more models and a second predicted colorants and masses thereof of the one or more predicted colorants and the masses thereof resulting from a second model of the one or more models.
Clause 21. The method of any of Clauses 18 to 20, wherein the method further comprises: collecting, after mixing the one or more colorants, a resultant liquid sample from the industrial mixing system and a batch acceptance status; analyzing the resultant liquid sample by the sensor to generate a resultant color measurement value associated with the resultant liquid sample; and transmitting the resultant color measurement value and the batch acceptance status to the color digitization processor.
Clause 22. The method of Clause 21, wherein executing the AI tinting prediction engine further comprises: updating the color digitization database with the resultant color measurement value; and re-generating the model selection policy for the target standard sample color measurement based on plurality of historical batch sample color information and the plurality of colorant information based on the batch color measurement value, the preferred predicted colorants and the masses thereof, the resultant color measurement value, and the batch acceptance status.
Clause 23. The method of Clause 21 or Clause 22, wherein the liquid batch comprises at least one of a paint batch, a food product batch, or a beverage product batch.

## Claims

1. A method of controlling a tinting of liquids in an industrial mixing system, the method comprising:
receiving, at a color digitization processor, a batch color measurement value associated with a liquid batch in an industrial mixing system;
executing, by the color digitization processor, an artificial intelligence, Al, tinting prediction engine, wherein executing the Al tinting prediction engine comprises:
storing the batch color measurement value in a color digitization database, the color digitization database comprising a plurality of historical batch sample color information, a plurality of target standard sample color measurement values, and a plurality of colorant information, wherein the colorant information includes a colorant value; and
modeling, by one or more models, tinting of the liquid batch by weighting one or more of the batch color measurement value, a batch volume, the plurality of historical batch sample color information, or the plurality of colorant information to generate one or more predicted colorants and masses thereof from the plurality of colorant information to achieve the target standard sample color measurement;
executing, by the color digitization processor, a virtual color sensor, wherein executing the virtual color sensor comprises:
performing virtual tinting based upon the batch color measurement value, the predicted colorants, and the masses thereof; and
generating a predicted color coordinate range based upon the virtual tinting;
executing, by the color digitization processor, a predicted weight accuracy engine, wherein executing the predicted weight accuracy engine comprises:
generating a perception boundary based on the plurality of historical batch sample color information and the plurality of target standard sample color measurement values, wherein the perception boundary comprises a plurality of color coordinates representing an outer boundary of accepted batches for the target standard sample color measurement value; and
determining a tinting confidence interval based upon the predicted color coordinate range and the perception boundary;
displaying, on a display coupled to the color digitization processor, the perception boundary, the predicted color coordinate range, and the confidence interval; and
providing, to the industrial mixing system, information relating to the predicted colorants and the masses thereof in response to an acceptance of the confidence interval, the predicted colorants and masses thereof for use in mixing, by the industrial mixing system, one or more colorants into the liquid batch.

2. The method of claim 1, wherein the one or more models comprise at least one of a linear model, a nonlinear model, a smart tinting model, a Gini index based model, or a near match model.

3. The method of claim 1 or claim 2, wherein executing the AI tinting prediction engine comprises:
generating a model selection policy for each of the plurality of the target standard sample color measurement values based on the plurality of historical batch sample color information; and
selecting a preferred model based on the model selection policy for the target standard sample color measurement value.

4. The method of any of claims 1 to 3, wherein the method comprises:
collecting, after mixing the one or more colorants, a resultant liquid sample from the industrial mixing system and a batch acceptance status;
analyzing the resultant liquid sample by the sensor to generate a resultant color measurement value associated with the resultant liquid sample; and
transmitting the resultant color measurement value and the batch acceptance status to the color digitization processor.

5. The method of claim 4, wherein executing the predicted weight accuracy engine comprises:
re-generating the perception boundary based on the plurality of historical batch sample color information, the plurality of target standard sample color measurement values, the resultant color measurement value, and the batch acceptance status.

6. The method of any of claims 1 to 5, wherein the method comprises:
sending the confidence interval to an insights processor wherein the insights processor is configured to transmit graphical data of the predicted color coordinate range, the perception boundary, and the confidence interval to a display.

7. The method of claim 6, wherein the method comprises:
receiving, after sending the confidence interval to the insights processor, an input of colorants and weights thereof;
re-executing, by the color digitization processor, the virtual color sensor, wherein re-executing the virtual color sensor comprises:
performing virtual tinting based upon the batch color measurement value, the input colorants, and the masses thereof; and
generating an updated predicted color coordinate range based upon the virtual tinting; and
re-executing, by the color digitization processor, the predicted weight accuracy engine, wherein re-executing the predicted weight accuracy engine comprises:
determining a tinting confidence interval based upon the updated predicted color coordinate range and the perception boundary.

8. The method of any of claims 1 to 7, wherein at least one of: the predicted color coordinate range comprises a CIELAB coordinate range or the perception boundary comprises a CIELAB coordinate range.

9. A liquid tinting system comprising:
a mixing system for mixing liquids;
a color digitization processor coupled to the mixing system;
a color digitization database coupled to the color digitization processor, the color digitization database storing colorant information and product information associated therewith; and
a memory storing computer-executable instructions that, when executed by the color digitization processor, configure the color tinting system for:
receiving liquid color information associated with a liquid batch;
identifying a product identifier based on the liquid color information and the product information; and
executing an artificial intelligence, Al, color prediction engine to predict colorants, wherein executing the Al color prediction engine comprises:
modeling, by one or more models, tinting of the liquid sample by predicting one or more colorants and colorant masses based on the liquid color information, the product identifier, and the colorant information;
generating a model selection policy for the product identifier based on the plurality of historical batch sample color information and product information;
selecting a preferred model of the one or more models based on the model selection policy for the product identifier; and
transmitting the colorants and colorant masses generated by the preferred model to the mixing system.

10. The system of claim 9, wherein the preferred model comprises a combination of a first model of the one or more models and a second model of the one or more models.

11. The system of claim 9 or claim 10, wherein the memory stores computer-executable instructions that, when executed by the color digitization processor, configure the color tinting system, after selecting a preferred model, for:
executing a virtual color sensor, wherein executing the virtual color sensor comprises:
performing virtual tinting based upon the liquid color information, the colorants and colorant masses generated by the preferred model; and
generating a predicted color coordinate range based upon the virtual tinting;
executing, by the color digitization processor, a predicted weight accuracy engine, wherein executing the predicted weight accuracy engine comprises:
generating a perception boundary based on liquid color information and the product information, wherein the perception boundary comprises a plurality of color coordinates representing an outer boundary of accepted batches for the target standard sample color measurement value; and
determining a tinting confidence interval based upon the predicted color coordinate range and the perception boundary; and
displaying, on a display coupled to the color digitization processor, the perception boundary, the predicted color coordinate range, and the confidence interval.

12. The system of any of claims 9 to 11, wherein the memory stores computer-executable instructions that, when executed by the color digitization processor, configure the color tinting system for:
receiving a result of mixing the colorants and colorant masses; and
re-executing the artificial intelligence, Al, color prediction engine to update the model selection policy, wherein re-executing the Al color prediction engine comprises:
updating the model selection policy for the product identifier based on the result of mixing the colorants and colorant masses, the plurality of historical batch sample color information, and product information.

13. A method of tinting liquids in an industrial mixing system, the method comprising:
receiving, by a color digitization processor, a batch color measurement value associated with a liquid batch in an industrial mixing system;
executing, by the color digitization processor, an artificial intelligence, Al, tinting prediction engine, wherein executing the Al tinting prediction engine comprises:
storing the batch color measurement value in a color digitization database, the color digitization database comprising a plurality of historical batch sample color information, a plurality of target standard sample color measurement values, and a plurality of colorant information wherein the colorant information includes a colorant value; and
modeling, by one or more models, tinting of the liquid batch by weighting one or more of the delta errors, the batch color measurement value, a batch volume, the plurality of historical batch sample color information, or the plurality of colorant information to generate one or more predicted colorants and masses thereof from the plurality of colorant information to achieve the target standard sample color measurement;
generating a model selection policy based on the plurality of historical batch sample color information and the target standard sample color measurement; and
selecting preferred predicted colorants and masses thereof by applying the model selection policy to the predicted colorants and masses thereof;
receiving, by the industrial mixing system, the preferred predicted colorants and the masses thereof; and
mixing, by the industrial mixing system, one or more colorants into the liquid batch based on the preferred predicted colorants and the masses thereof.

14. The method of claim 13, wherein applying the model selection policy to the predicted colorants and masses thereof comprises:
applying the model selection policy to the predicted colorants and masses thereof by combining a first predicted colorants and masses thereof of the one or more predicted colorants and the masses thereof resulting from a first model of the one or more models and a second predicted colorants and masses thereof of the one or more predicted colorants and the masses thereof resulting from a second model of the one or more models.

15. The method of claim 13 or claim 14, wherein the method comprises:
collecting, after mixing the one or more colorants, a resultant liquid sample from the industrial mixing system and a batch acceptance status;
analyzing the resultant liquid sample by the sensor to generate a resultant color measurement value associated with the resultant liquid sample; and
transmitting the resultant color measurement value and the batch acceptance status to the color digitization processor;
and optionally:
executing the Al tinting prediction engine comprises:
updating the color digitization database with the resultant color measurement value; and
re-generating the model selection policy for the target standard sample color measurement based on plurality of historical batch sample color information and the plurality of colorant information based on the batch color measurement value, the preferred predicted colorants and the masses thereof, the resultant color measurement value, and the batch acceptance status.
